# EUROPÄISCHE PATENTSCHRIFT

(11) **EP 0 706 367 B1**
(45) Veröffentlichungstag und Bekanntmachung des Hinweises auf die Patenterteilung: **22.01.1997**
(21) Anmeldenummer: 94920378.0
(22) Anmeldetag: 28.06.1994
(51) Int. Cl.: A61K 7/48, A61K 7/50

(54) **DUSCHLOTIONEN ZUR BEHANDLUNG UND PFLEGE TROCKENER HAUT**
SHOWER LOTIONS FOR DRY SKIN TREATMENT AND CARE
LOTIONS DE DOUCHE POUR LE TRAITEMENT ET LES SOINS DES PEAUX SECHES

(30) Priorität: 01.07.1993 DE 4322395
(43) Veröffentlichungstag der Anmeldung: 17.04.1996
(73) Patentinhaber: Kühnl-Petzoldt, Christa, D-79249 Merzhausen (DE); Kownatzki, Eckhard, D-79249 Merzhausen (DE)
(72) Erfinder: Kühnl-Petzoldt, Christa, D-79249 Merzhausen (DE); Kownatzki, Eckhard, D-79249 Merzhausen (DE)
(74) Vertreter: Maikowski, Michael, Dipl.-Ing. Dr.
(86) Internationale Anmeldenummer: DE9400772
(87) Internationale Veröffentlichungsnummer: WO9501158

(56) Entgegenhaltungen:
- EP-A- 0 105 657
- EP-A- 0 158 108
- EP-A- 0 256 821
- EP-A- 0 293 503
- WO-A-87/04617
- AU-B- 1 205 170
- DE-A- 2 725 525
- DE-A- 2 913 040
- GB-A- 2 092 444
- GB-A- 2 226 256
- S.T.N., Datenbank Lifferant, Karlsruhe, DE Datenbank Chemical Abstracts, Band 118, N 154177 siehe die Zusammenfassung
- DATABASE WPI Week 8721, Derwent Publications Ltd., London, GB; AN 87-145358 & HU,A,41 255 (PLANORG MERNOKI)
- PATENT ABSTRACTS OF JAPAN vol. 11, no. 167 (C-425) (2614) 28. Mai 1987 & JP,A,62 000 007 (SHISEIDO)

## Beschreibung

Die Erfindung betrifft nichtschäumende, die Haut nicht entfettende Duschlotionen zur Behandlung und Pflege normaler und trockener Haut.

Zahlreiche Personen besitzen eine empfindliche Haut, die beim Reinigen mit Körperreinigungsmitteln austrocknet. Durch häufiges Baden, Duschen oder Waschen unter Anwendung von Seife oder Detergentien wird der Film von hauteigenem Fett und fettähnlichen Substanzen zerstört. Ist der natürliche Fettfilm zerstört, kann Wasser die Haut angreifen und vorteilhafte wasserlösliche Hautbestandteile ausspülen. Dazu gehört Harnstoff als der wichtigste natürliche Feuchthalter. Die Folge ist eine trockene, rissige, spröde Haut.

Trockene, rissige, spröde Haut und ihre Folgeerscheinungen treten bei etwa der Hälfte aller Kinder und Heranwachsenden auf. Im Alter steigt dieser Anteil auf über 70 %.

Bei Menschen mit trockener Haut sind erniedrigte Harnstoffkonzentrationen in der Hornschicht nachgewiesen worden.

Zur Therapie der trockenen Haut werden seit vielen Jahren Cremes mit einem Harnstoffgehalt von 2 bis 10 % eingesetzt. Eine Behandlung mit einer harnstoffhaltigen Creme soll der ausgelaugten Haut ihre natürliche Wasserbindungsfähigkeit zurückgeben und den zerstörten Fettfilm wieder herstellen.

Der partielle Ersatz zuvor ausgewaschener Hautbestandteile durch Cremes ist jedoch mit Nachteilen verbunden.

Das Eincremen ersetzt nur unvollständig die Schutzstoffe, die der Haut zuvor durch die Reinigung mit Wasser und Seife entzogen wurden. Die Harnstoffzufuhr ist mit Cremes nur beschränkt möglich.

Die Konzentration an Harnstoff soll nach Empfehlung von Hautärzten 10 % nicht überschreiten, da es sonst zu Hautreizungen kommt. Außerdem neigen hochkonzentrierte Harnstoffcremes zum Auskristallisieren. In wässrigen Lösungen zerfällt Harnstoff leicht und erzeugt dann einen unangenehm stechenden Geruch. Konservierungsstoffe, die ein Bakterienwachstum verhindern sollen, rufen ihrerseits Unverträglichkeitsreaktionen der Haut hervor.

Eine von außen zugeführte Fettschicht, die auf der Haut verbleibt, wird als unangenehm fettig oder schmierig empfunden. Das Einbalsamieren der gesamten Oberfläche des menschlichen Körpers ist zeitaufwendig und wird oft als mühsam und lästig empfunden.

Aus der französischen Patentschrift 2 498 929 ist eine Feuchtigkeitsemulsion in Form einer Öl-in-Wasser-Emulsion bekannt, die neben den üblichen Inhaltsstoffen Glycerin und Fetten auch Harnstoff enthält. Die Harnstoffkonzentration in dieser Emulsion liegt dabei in dem von Hautärzten empfohlenen Bereich von maximal 12,5 %. Außerdem enthält diese Emulsion zur Stabilisierung Konservierungsstoffe.

Mit der vorgegebenen Zusammensetzung erfüllt die Emulsion nicht die Kriterien, die an den Einsatz von nichtschäumenden Duschlotionen zur Behandlung und Pflege trockener Haut gestellt werden.

Der Erfindung liegt die Aufgabe zugrunde, nichtschäumende, die Haut nicht entfettende Duschlotionen zur Behandlung und Pflege trockener Haut zu entwickeln, die ohne den Zusatz von Konservierungsstoffen über einen langen Zeitraum stabil bleiben, eine Reinigung ohne Detergentienzusatz ermöglichen und unter Vermeidung der Austrocknung durch häufiges Waschen zu einer geschmeidigen Hautoberfläche führen.

Erfindungsgemäß wird die Aufgabe durch Duschlotionen gemäß der kennzeichnenden Merkmale der Ansprüche gelöst.

Die erfindungsgemäße nichtschäumende, die Haut nicht entfettende Duschlotion zur Behandlung trockener Haut besteht aus einer Wasser, Harnstoff, Glycerin und übliche Zusatzstoffe enthaltenden Öl-in-Wasser-Emulsion. Sie enthält 15 bis 35 Gew.% Harnstoff, 20 bis 60 Gew.% Glycerin, 5 bis 20 Gew.% üblicher Zusatzstoffe aus der Gruppe der Fette bzw. fettähnlichen Substanzen, wie Vaseline und Paraffine, Lösungsvermittler, Duftstoffe und Emulgatoren und als Rest Wasser zu 100%.

Besonders geeignet sind Mengen an Glycerin von 25 - 35 Gew. % .

Das Glycerin wirkt als Lösungsvermittler und verhindert das Auskristallisieren des Harnstoffs.

Als übliche Zusatzstoffe kommen u.a. Fette bzw. fettähnliche Substanzen, wie Vaseline oder Paraffine, Lösungsvermittler, Duftstoffe und Emulgatoren, aber keine Konservierungsmittel zum Einsatz.

Vorzugsweise enthält die Duschlotion 25 bis 30 Gew.% Harnstoff, 25 bis 30 Gew.% Glycerin, 5 bis 10 Gew.% übliche Zusatzstoffe und als Rest Wasser.

Ganz vorzugsweise enthält die Duschlotion 30 Gew.% Harnstoff, 30 Gew.% Glycerin, 10 Gew.% übliche Zusatzstoffe und 30 Gew.% Wasser.

Mit den erfindungsgemäßen Duschlotionen als kombinierte Reinigungs- und Pflegelotionen, welche den natürlichen Fettfilm der Haut nicht zerstören und daneben zu einer Hydratation und Glättung der Haut führen, werden schnellwirkende Mittel zur Verfügung gestellt.

Die Duschlotionen werden auf die angefeuchtete Haut aufgetragen und nach einer Einwirkzeit von 30 bis 90 Sekunden abgespült. Schon in dieser kurzen Zeitspanne bewirkt der hochkonzentrierte Harnstoff eine Reinigung, da Harnstoff in hohen Konzentrationen Verbindungen zwischen der Haut und unhaftenden Schnutzteilchen löst. Der körpereigene Fettfilm wird dabei kaum angegriffen.

Die Harnstoffkonzentration der erfindungsgemäßen Duschlotionen beträgt etwa das 20fache der Konzentration in der gesunden Haut und etwa das 100fache der Konzentration in einer harnstoffverarmten Haut. Der hohe Überschuß der Konzentration an Harnstoff in den Duschlotionen bewirkt insbesondere bei einer harnstoffverarmten Haut eine schnelle Diffusion des Harnstoffes in die Hornschicht. Die Einwirkzeit der Duschlotionen ist nur kurz. Da die Duschlotionen dann abgewaschen werden, kommt es wegen der hohen Konzentration an Harnstoff zwar zu einer Aufnahme durch die Haut, nicht aber zu Hautreizungen.

Die hohe Konzentration an Glycerin in den Duschlotionen verhindert ein Auskristallisieren ebenso wie eine Zersetzung des Harnstoffs. Die hohe Konzentration an Harnstoff in den Duschlotionen hemmt zusätzlich das Wachstum von Bakterien. Der Einsatz von Konservierungsmitteln in den Duschlotionen wird unnötig.

Die Kombination der Inhaltsstoffe der Duschlotionen führt zu einem Körperbehandlungsmittel, das bei regelmäßiger Anwendung bereits nach kurzer Zeit zu einem angenehmen, samtigen Erscheinungsbild der Haut führt. Die wirksame Hautreinigung wird ohne Seife und künstliche Detergentien erreicht.

Der Einsatz von Glycerin, sowohl als Lösungsvermittler als auch als Stabilisator für den Harnstoff, macht die Duschlotion trotz hoher Konzentration an Harnstoff über einen langen Zeitraum stabil.

Zur Herstellung der Duschlotion werden drei Teile Glycerin und drei Teile Wasser miteinander gemischt und auf 80° C erhitzt. Nach Erwärmung und Durchmischung werden der heißen Mischung unter Aufrechterhaltung der Temperatur drei Teile Harnstoff hinzugefügt und so lange gerührt, bis sich der Harnstoff aufgelöst hat. Anschließend wird ein Teil eines auf 80° C vorgewärmten homogen gemischten Fettes hinzugefügt und die Mischung mittels Rührgerät emulgiert. Die so erhaltene Emulsion bildet die fertige Duschlotion .

## Patentansprüche

1. Nichtschäumende, die Haut nicht entfettende Duschlotionen zur Behandlung und Pflege trockener Haut, **gekennzeichnet durch** eine Öl-in-Wasser-Emulsion, die
15 bis 35 Gew-% Harnstoff,
20 bis 60 Gew-% Glycerin,
5 bis 20 Gew-% üblicher Zusatzstoffe
aus der Gruppe der Fette bzw. fettähnlichen Substanzen, wie Vaseline und Paraffine, Lösungsvermittler, Duftstoffe und Emulgatoren
und Wasser als Rest zu 100 %
enthält.

2. Duschlotionen nach Anspruch 1, **gekennzeichnet durch** einen Gehalt an Glycerin in Mengen von 25 - 35 Gew.%.

3. Duschlotion nach Anspruch 1 und 2, **gekennzeichnet durch** eine Öl-in-Wasser-Emulsion, die
30 Gew-% Harnstoff,
30 Gew-% Glycerin,
10 Gew-% üblicher Zusatzstoffe,
30 Gew-% Wasser
enthält.

## Claims

1. Non-foaming, non-delipidating shower lotions for the treatment and care of dry skin, **characterised in that** an oil-in-water-emulsion contains
15 to 35 weight-% urea,
20 to 60 weight-% glycerin,
5 to 20 weight-% usual additives
being members of the fat group or respectively fatlike substances like vaseline and paraffins, solubilizers, fragrances and emulgators,
and water to make up 100%.

2. Shower lotions according to claim 1 **characterised in that** they contain glycerin in the concentration range of 25 to 35 weight-%.

3. Shower lotion according to claims 1 and 2, **characterised in that** an oil-in-water-emulsion contains
30 weight-% urea,
30 weight-% glycerin,
10 weight-% usual additives,
30 weight-% water.

## Revendications

1. Lotions de douche non moussantes, qui sont destinées au traitement et aux soins de peaux sèches et qui ne dégraissent pas celles-ci, caractérisées par une émulsion huile dans eau, qui contient
15 à 35 % en poids d'urée,
20 à 60 % en poids de glycérine,
5 à 20 % en poids d'additifs courants du groupe des graisses et respectivement substances analogues à des graisses, comme des vaselines et des paraffines, des tiers-solvants, des parfums et des agents émulsionnants,
le reste étant de l'eau pour former 100 %.

2. Lotions de douche suivant la revendication 1, caractérisées par une teneur en glycérine en des quantités de 25 à 35 % en poids.

3. Lotions de douche suivant l'une des revendications 1 et 2, caractérisées par une émulsion huile dans eau qui contient
30 % en poids d'urée,
30 % en poids de glycérine,
10 % en poids d'additifs courants,
30 % en poids d'eau.
